Europäisches Patentamt

European Patent Office

Office européen des brevets

(11) Veröffentlichungsnummer: **0 224 837 B1**

# (12) EUROPÄISCHE PATENTSCHRIFT

(45) Veröffentlichungstag der Patentschrift: **23.12.92**

(51) Int. Cl.5: **A61K 9/50**, A61K 7/00

(21) Anmeldenummer: **86116286.5**

(22) Anmeldetag: **24.11.86**

Die Akte enthält technische Angaben, die nach dem Eingang der Anmeldung eingereicht wurden und die nicht in dieser Patentschrift enthalten sind.

(54) Hautwirksame Pharmaka mit Liposomen als Wirkstoffträger.

(30) Priorität: **04.12.85 DE 3542773**

(43) Veröffentlichungstag der Anmeldung:
**10.06.87 Patentblatt 87/24**

(45) Bekanntmachung des Hinweises auf die Patenterteilung:
**23.12.92 Patentblatt 92/52**

(84) Benannte Vertragsstaaten:
**AT CH DE FR GB IT LI NL**

(56) Entgegenhaltungen:
**US-A- 3 957 971**

**PHARMAZIE, Band 39, Nr. 9, September 1984, Seiten 627-629, Ost-Berlin, DD; L. KROWCZYNSKI et al.: "Liposomen als Wirkstoffträger in der percutanen Therapie"**

**CHEMICAL ABSTRACTS, Band 93, Nr. 2, 14. Juli 1980, Seite 314, Zusammenfassung Nr. 13043m, Columbus, Ohio, US; M. MEZEI et al.: "Liposomes - a selective drug delivery system for the topical route of administration. I. Lotion dosage form", & LIFE SCI. 1980, 26(18), 1473-7**

**CHEMICAL ABSTRACTS, Band 80, Nr. 5, 4. Februar 1974, Seite 45, Zusammenfassung Nr. 22841d, Columbus, Ohio, US; L.B. FISHER et al.: "Topical antipsoriatic agents and epidermal mitosis in man", & ARCH. DERMATOL. 1973, 108(3), 374-7**

**ROTE LISTE, 1983, Editio Cantor, Aulendorf/Württ., DE**

(73) Patentinhaber: **Röhm Pharma GmbH
Dr.-Otto-Röhm-Strasse 2-4
W-6108 Weiterstadt 1(DE)**

(72) Erfinder: **Müller, Josef, Dr. Dipl.-Chem.
Jugendpfad 5
W-6145 Lindenfels(DE)**

EP 0 224 837 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Medikamente zur Therapie von Erkrankungen der Haut, die gegebenenfalls schon an sich für die Behandlung von Hautkrankheiten bekannte Wirkstoffe enthalten, und die als Externa im Bereich der erkrankten Hautstelle angewendet werden.

Zur Lokaltherapie von Hautkrankheiten werden in der Regel Salben, Cremes, Lotionen oder Tinkturen verwendet, in die bestimmte Wirkstoffe eingearbeitet sind. Für den therapeutischen Effekt dieser Wirkstoffe ist Voraussetzung, daß sie aus der entsprechenden Grundlage in ausreichender Menge freigegeben werden. Nur wenn dies der Fall ist, können die Wirkstoffe dann in die Haut eindringen und dorthin gelangen, wo ihre Wirkungsqualitäten benötigt werden.

Neuartige Arzneistoffträger sind die Liposomen. Als solche werden kugelförmige Gebilde aus einer oder mehreren Lipiddoppelschichten mit wässrigen Innenraum bezeichnet, und die sich aus Phospholipiden, z. B. Lecithin, unter anderem durch deren mechanische Feinverteilung herstellen lassen. Herstellungsweisen von Liposomen und deren Verwendung als Arzneimittel- oder Kosmetikmittelträger, wobei die Wirkstoffe in den Innenräumen der Liposomen eingelagert sind, sind in US-A 3 957 971, in DE-A 28 18 655, in DE-A 28 34 308 oder in GB-A-2 013 609 angegeben. Aus "Pharmazie in unserer Zeit" 11, (1982) 97 bis 108, ist praktisch der derzeitige Stand des Wissens bezüglich Herstellung von Liposomen und deren Anwendung als Arzneimittelträger ersichtlich. Danach sind Applikationsarten wie intravenöse, intramuskuläre und subcutane Injektionen und auch orale Verabreichungen von arzneimittelhaltigen Liposomen getestet worden. Prüfungen von medikamentösen Formulierungen mit Liposomen als Träger zu lokalen Anwendungen auf der Haut, z. B. bei Hautkrankheiten, standen möglicherweise Vorurteile infolge der Kenntnisse des Hautaufbaues und die vielfältigen Erfahrungen mit Applikationen auf der Haut entgegen, da in der Epidermis unter dem Stratum corneum, der die Oberfläche der Haut bildenden Hornschicht, eine dichter gebaute Zellage, das Stratum conjunctum oder compactum, liegt, deren Funktion die Erschwerung bzw. Verhinderung der Penetration von Stoffen von außen ist.

Auch Pharmazie, Bd. 39, 9/1984, 627-629, beschreibt "Liposomen als Wirkstoffträger in der percutanen Therapie", für Triamcinolon als Modellsubstanz mit drei- bis viermal höherer Resorption percutan.

Die am häufigsten verwendeten Wirkstoffe zur äußeren Behandlung von Hautkrankheiten sind Kortikosteroide und Trihydroxianthracene. So beschreibt die Rote Liste 1983 unter Nr. 31 370 Psoradexan®-Creme (Dithranol/Harnstoff) zur Anwendung bei subakuter und chronischer Psoriasis. Wegen ihres geringen Penetrationsvermögens durch die Haut nach Liberation aus den Trägergrundlagen, müssen diese Stoffe, um einen ausreichenden therapeutischen Effekt zu erzielen in hoher Konzentration und in hohem Überschuß in z. B. Salben oder Cremes vorliegen.

## Aufgabe und Gegenstand der Erfindung

Es war daher Aufgabe der Erfindung hautwirksame Pharmaka zu finden, mit denen die Wirkstoffe schneller in die Haut dringen bzw. diese durchdringen können, um so Medikamente zur Verfügung zu haben, die die Wirkungssubstanzen ständig und in ausreichender Menge an den Wirkort geben.

Hautwirksame Stoffe, wie z. B. Kortikosteroide oder Anthracentriole, lassen sich relativ einfach mit Liposomen kombinieren, d. h. in diese einschließen. Überraschenderweise wurde gefunden, daß mit so beladenen Liposomen, in einer streichfähigen Form z. B. als Salben oder Cremes auf die Haut gebracht, die Penetration der Wirkstoffe wesentlich schneller erfolgt als im Vergleich aus einer üblichen Wirkstoff-Salben-Formulierung.

Wie weiter gefunden wurde, läßt sich Harnstoff, von dem zur Therapie von Hauterkrankungen selbst eine ganze Reihe von Wirkungsqualitäten, z. B. als Keratolytikum, bekannt sind, vorteilhaft mit in die Liposomenpräparate ein arbeiten, wobei Mischungen von Harnstoff und Hydroxyanthracenen (Anthratriole) und gegebenfalls weiteren hautwirksamen Stoffen, wie z. B. Steroiden mit den Liposomen bzw. den Phospholipidgemischen als deren Vorstadium zu hautwirksamen Medikamenten formuliert werden.

Beispielsweise werden mit erfindungsgemäßen Harnstoff-Dithranol-Zubereitungen etwa 3 bis 4 mal höhere Wirkstoff-Haut Penetrationen als mit bisher üblichen Formulierungen gemessen. Somit ermöglichen die neuen Formulierungen erhebliche Einsparungen an Wirkstoffmengen. Durch die Mengeneinsparung werden auch andere Probleme, wie z. B. Verschmutzung von Textilien, die insbesondere bei Anwendung farbiger Substanzen oder farbiger Metabolite, wie z. B. Oxidationsprodukte der Anthratriole, auftreten, entschärft bzw. gelöst.

## Durchführung der Erfindung

Die Herstellung der erfindungsgemäß anzuwendenden Liposomenzubereitung aus den Aufbaukomponenten der Liposomen, den Phospholipiden, insbesondere den Phosphatidylcholinen, wie den Lecithinen, einer in der Natur vorkommenden Gruppe von Phospholipiden, beispielsweise Lecithin aus Sojabohnen, dem gebräuchlichsten Rohstoff zur Herstellung von Lecithin, sowie weiteren Zusatzstoffen, wie Sterinen, z. B. dem Cholsterin, und den hautwirksamen Stoffen, dem Harnstoff und den Hydroxyanthracenen, kann nach verschiedenen Methoden, wie sie beispielsweise in den oben angegebenen Literaturstellen beschrieben sind, vorgenommen werden. Das für die Herstellung der Liposomenträger anzuwendende Verhältnis der in der Lipidausgangsmischung vorhandenen Substanzen, von z. B. Lecithin zu z. B. Cholesterin, kann in den Bereichen 10 : 0,1 bis etwa 1 : 1, vorwiedend bei 10 : 1 bis 1 : 1, insbesondere bei 5 : 1 bis 2 : 1 liegen. Auch weitere Substanzen, z. B. Dialkylphosphate oder Sphingomyelin oder Hilfsstoffe wie Zucker- bzw. Aminozuckerderivate, z. B. zur Stabilisierung der Liposomenmembran, und Antioxidantien können bei der Präparierung mitverwendet werden.

Die Herstellung kann beispielsweise nach der bekannten Film-Dispersions-Methode, einer schonenden Eindampfung, z. B. im Rotationsverdampfer, der Lipid-Wirkstoff-Mischung in Chloroform-Alkohol-Lösung vorgenommen werden, welcher zur Herstellung besonders kleiner Partikel mit relativ enger Größenverteilung eine Ultraschallbehandlung nachgeschaltet werden kann. Durch Zentrifugieren und Waschen können die wirkstoffhaltigen Liposomen dann isoliert und gereinigt werden. Die Partikelgröße der nach solchen Verfahren erhaltenen Liposomen kann stark variieren und im Bereich von einem oder mehreren Micrometern bis hinunter zu etwa 10 nm liegen. Für die Verwendung als hautwirksame Pharmakaträger haben die Liposomen vorzugsweise Durchmesser von 20 bis 50 nm.

Wirkungssubstanzen, die mit den obengenannten Aufbaukomponenten der Liposomen erfindungsgemäße hautwirksame Pharmaka bilden, gehören z. B. der großen Gruppen der Antibiotika oder der Sulfonamide an, oder sind insbesondere solche aus der Gruppe der Kortikosteroide und Hydroxyanthracene. Die therapeutischen Wirkungen dieser Stoffe sind bekanntermaßen insbesondere bei der Behandlung entzündlicher, ekzematöser oder allergischer Hauterkrankungen angezeigt. Durch Kombination von Wirkstoffen aus den verschiedenen Substanzgruppen wird noch eine therapeutische Wirkungssteigerung erzielt. Die Verwendung von Harnstoff bei der Herstellung wirkstoffhaltiger Liposomen in Kombination mit Hydroxyanthracenen und gegebenfalls weiter mit Kortikosteroiden, führt zu therapeutisch wertvollen Formulierungen. Die Vorteile der erfindungsgemäßen Medikamente gegenüber bekannten Medikamenten mit gleichen Wirksubstanzen, sind durch die verbesserte Penetration durch die Haut, damit einer höheren Verfügbarkeit des Arzneistoffes am Wirkort und somit einer deutlichen Senkung der anzuwendenden Wirkstoffmenge gegeben, was sich in einer Verringerung von Nebenwirkungen und wesentlichen Verbilligung des Medikaments niederschlägt.

Erfindungsgemäß einsetzbare Kortikosteroide sind beispielsweise Fludrocortison, Fluocortolon, Fluorandrenolon, Triamcinolon, Methylprednisolon; Anthracentriole, die in den erfindungsgemäßen Formulierungen eingesetzt werden, und die als Dermatica oder Antiseptika bekannt sind, sind die Hydroxyanthracene 1,8,9-Anthracentriol, auch als 1,8,9-Anthratriol bzw. 1,8-Dihydroxianthranol-(9) und unter dem von der WHO vorgeschlagenen Freinamen Dithranol bekannt, oder 1,2,10-Anthracentriol, das auch als 1,2,10-Anthratriol bekannt ist.

Auch für die transdermale Applikation von Wirkstoffen verschiedener Art bei Erkrankungen anderer Organismusteile als der Haut bzw. hautnaher Bereiche, ist die Verwendung von Medikamenten mit pharmakahaltigen Liposomen wegen deren besseren Hautpenetration und damit schnellerem Transport der Pharmaka deutlich wirkungsvoller.

Die Zubereitungen können auch noch Wirkstoffe für die beispielsweise eine Hautpenetration nicht angezeigt oder deren Penetrationsgeschwindigkeit nicht so kritisch ist, in bisher üblicher Zugabe enthalten.

Die zur äußeren Anwendung an der Haut hergestellten Zubereitungen sind disperse Systeme, die die Wirkstoff-Liposomen-Präparationen und gegebenenfalls weitere Wirkstoffe, gelöst, emulgiert oder suspendiert enthalten. Die Applikationsart der neuartigen hautwirksamen Medikamente mit pharmakahaltigen Liposomen wird im wesentlichen nach bisher bekannter Vorgehensweisen, d. h. beispielsweise als Salben oder Cremes durchgeführt.

Experimenteller Teil

1. Herstellung von Liposomen-Wirkstoff-Kombinationen

Chloroform/Äthanol 1 : 1 - oder Chloroform/Methanol 1 : 1 - Lösungen von Lecithin, Cholesterin und Wirkstoff, werden bei etwa 30°C im Rotationsverdampfer vom Lösungsmittel unter Bildung eines dünnen Lipid-Wirkstoff-Films befreit. Anschließend wird der Film mit 8 molarer Calciumchloridlösung von 60°C versetzt und durch manuelles Schütteln die Ablösung der Liposomen von der Wandung erwirkt. Die so erhaltene Dispersion wird dann im Ultraschalldesintegrator weiter zerkleinert, anschließend zentrifugiert und dann die Liposomen-Wirkstoff-Kombination dreimal mit Calciumchloridlösung gewaschen.

Aus der folgenden Zusammenstellung sind die Zusammensetzungen hergestellter Liposomen in Gewichtsteilen Lecithin und Cholesterin und die von den eingesetzten Wirkstoffen Triamcinolon oder Dithranol eingebauten Anteile ersichtlich.

| Lecithin (Gewichtsteile) | | 30 | 30 | 32.5 | 32.5 | 35 | 35 |
|---|---|---|---|---|---|---|---|
| Cholesterin (Gewichtsteile) | | 13 | 10 | 13 | 10 | 10 | 7 |
| Einbau von eingesetztem | Triamcinolon (in %) oder | 41 | 32.4 | 56.2 | 42.8 | 62.1 | 49.8 |
| | Dithranol (in %) | 69.7 | 42.1 | 85.6 | 76.1 | 87.5 | 62.1 |

**Patentansprüche**

1. Hautwirksame, pharmazeutische Zubereitung zur äußeren Anwendung auf der Haut, die im wesentlichen aus Harnstoff und mindestens einem Wirkstoff aus der Gruppe der Hydroxyanthracene besteht,
   dadurch gekennzeichnet,
   daß die Harnstoff-Hydroxyanthracen-Mischung in Liposomen eingearbeitet bzw. eingeschlossen ist.

2. Hautwirksame, pharmazeutische Zubereitung nach Anspruch 1, dadurch gekennzeichnet, daß das Hydroxyanthracen Dithranol ist.

3. Hautwirksame, pharmazeutische Zubereitung nach den Ansprüchen 1 und 2, dadurch gekennzeichnet, daß die Zubereitung neben der Harnstoff-Hydroxyanthracen-Mischung noch weitere bekannte hautwirksame Wirkstoffe in der Liposomen-Einarbeitung enthält.

4. Hautwirksame, pharmazeutische Zubereitung nach den Ansprüchen 1 bis 3, dadurch gekennzeichnet, daß der Zubereitung mit den in Liposomen eingeschlossenen Wirkstoffen auch Wirkstoffe in bisher üblicher Zubereitung beigegeben sind.

**Claims**

1. A dermatologically effective, pharmaceutical preparation for external use on the skin consisting largely of urea and at least one active substance from the group of hydroxyanthracenes, characterised in that the urea-hydroxyanthracene mixture is incorporated or enclosed in the liposomes.

2. A dermatologically effective, pharmaceutical preparation according to claim 1, characterised in that the hydroxyanthracene is dithranol.

3. A dermatologically effective, pharmaceutical preparation according to claims 1 and 2, characterised in that the preparation also has, apart from the urea-hydroxyanthracene mixture, further known dermatologically effective active substances which are incorporated in the liposomes.

4. A dermatologically effective, pharmaceutical preparation according to claims 1 to 3, characterised in that active substances as used in a hitherto conventional preparation are also added to the preparation containing active substances enclosed in the liposomes.

**Revendications**

1. Préparation pharmaceutique à action cutanée pour usage externe sur la peau, se composant essentiellement d'urée et d'au moins une substance active du groupe des hydroxyanthracènes, caractérisée en ce que le mélange urée/hydroxyanthracène est incorporé ou inclus dans des liposomes.

2. Préparation pharmaceutique à action cutanée selon la revendication 1, caractérisée en ce que l'hydroxyanthracène est le dithranol.

3. Préparation pharmaceutique à action cutanée selon la revendication 1 ou 2, caractérisée en ce qu'elle contient, outre le mélange urée/hydroxyanthracène, d'autres substances actives connues à action cutanée, incorporées dans les liposomes.

4. Préparation pharmaceutique à action cutanée selon l'une quelconque des revendications 1 à 4, caractérisée en ce qu'il est adjoint, à la préparation contenant les substances actives incluses dans des liposomes, des substances actives en préparation jusqu'ici classique.